# EUROPEAN PATENT SPECIFICATION

(11) **EP 2 038 260 B1**
(45) Date of publication and mention of the grant of the patent: **01.09.2010**
(21) Application number: 07730171.1
(22) Date of filing: 14.06.2007
(51) Int. Cl.: C07D 241/04

(54) **STEREOSELECTIVE SYNTHESIS OF (S)-1-METHYL-3-PHENYLPIPERAZINE**
STEREOSELEKTIVE SYNTHESE VON (S)-1-METHYL-3-PHENYLPIPERAZIN
SYNTHÈSE STÉRÉOSÉLECTIVE DE (S)-1-MÉTHYL-3-PHÉNYLPIPÉRAZINE

(30) Priority: 16.06.2006 EP 06115607
(43) Date of publication of application: 25.03.2009
(73) Proprietor: N.V. Organon, 5349 AB Oss (NL)
(72) Inventor: VAN VLIET, Michiel, Christian, Alexander, 2611 VC Delft (NL); KEMPERMAN, Gerardus Johannes, 5340 BH Oss (NL); SCHREUDER GOEDHEIJT, Marcel, 5340 BH Oss (NL)
(74) Representative: Broekkamp, Chris L. E.
(86) International application number: PCT/EP2007/055914
(87) International publication number: WO 2007/144409

(56) References cited:
- EP-A- 1 426 356
- US-A1- 2004 242 879
- BREEN G F: "Enzymatic resolution of a secondary amine using novel acylating reagents" TETRAHEDRON: ASYMMETRY, ELSEVIER SCIENCE PUBLISHERS, AMSTERDAM, NL, vol. 15, no. 9, 10 May 2004 (2004-05-10), pages 1427-1430, XP004505346 ISSN: 0957-4166 cited in the application
- HU S ET AL: "An Efficient and Practical Chemoenzymatic Preparation of Optically Active Secondary Amines" ORGANIC LETTERS, ACS, WASHINGTON, DC, US, vol. 7, no. 20, 27 August 2005 (2005-08-27), pages 4329-4331, XP002395193 ISSN: 1523-7060 cited in the application
- MORGAN B ET AL: "Enzymatic Kinetic Resolution of Piperidine Atropisomers: Synthesis of a Key Intermediate of the Farnesyl Protein Transferase Inhibitor SCH66336" JOURNAL OF ORGANIC CHEMISTRY, AMERICAN CHEMICAL SOCIETY. EASTON, US, vol. 65, no. 18, 8 September 2000 (2000-09-08), pages 5451-5459, XP002395194 ISSN: 0022-3263 cited in the application
- ORSAT B ET AL: "Homocarbonates as Substrates for the Enantioselective Enzymatic Protection of Amines" JOURNAL OF THE AMERICAN CHEMICAL SOCIETY, AMERICAN CHEMICAL SOCIETY, WASHINGTON, DC, US, vol. 118, no. 3, 1996, pages 712-713, XP002395195 ISSN: 0002-7863 cited in the application

## Description

This invention relates to a novel starting material and the use thereof in a method to prepare (S)-1-methyl-3-phenylpiperazine or (R)-1-methyl-3-phenylpiperazine by enzymatic hydrolysis of an ester of racemic 1-methyl-3-phenylpiperazine.

In order to obtain optically active starting material 1-methyl-3-phenylpiparazine for the stereoselective synthesis route towards S-mirtazapine (See Wieringa et al, WO2005/005410) there is a need for an efficient method of preparation of (S)-1-methyl-3-phenylpiperazine of high enantiomeric purity. Biocatalysis is an excellent tool for preparation of optically active compounds. Enzymes often display chemo, enantio- and regio-selectivity under very mild conditions. Furthermore, biocatalysis allows for the use of methods that have few equivalents in organic chemistry. An example of this is consuming the undesired enantiomer with moderate enantioselectivity up to 99% ee (enantiomeric excess) is reached at the expense of some yield. Biocatalysis is not always absolutely selective. Good results can be obtained, though, by proper selection of an enzyme followed by an optimization that requires understanding of the underlying mechanism. The final result can be less complicated than a standard chemical process.

Attempts for enantioselective, enzymatic acylation of *rac*-1-methyl-3-phenylpiperazine according to scheme 1 failed as there was no observable reaction at temperatures up to 55 °C using the very reactive trifluoroethyl butyrate and large amounts of various enzymes, despite published examples of successful enantioselective acylation, with a well-balanced combination of enzyme and acyl donor (Orsat, et al, J. Am. Chem. Soc. 1996 (118) 712.; Morgan et al.; J. Org. Chem. 2000 (65) 5451; Breen, Tetrahedron: Asymmetry 2004 (15) 1427).

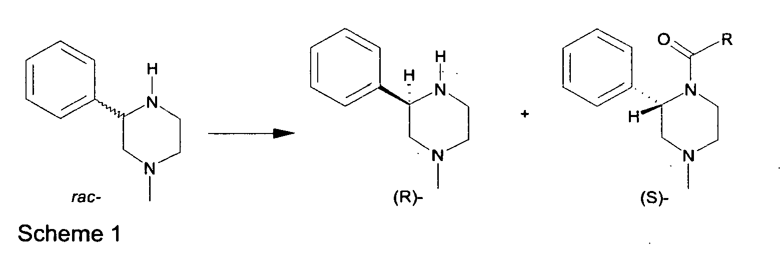

It was found by Hu et al (Org. Lett. 2005 (7) 4329) that resolution of secondary amines, using the enzymatic hydrolysis of an oxalamate group was feasible (Scheme 2). After separation of the ester and acid products and cleavage of the oxalamic groups both enantiomers of the amines can be obtained. The unique feature is the use of a remote oxalamate ester group for this resolution, while the low-reactive amide bond is not converted.

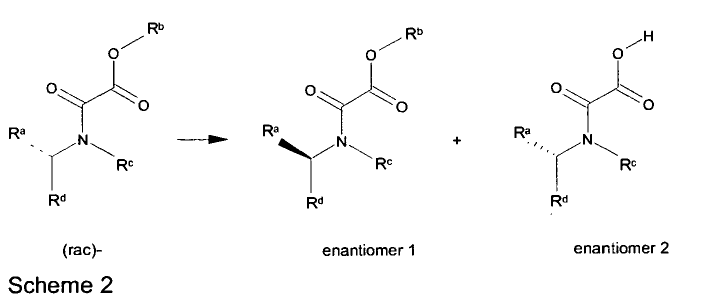

This invention provides for a compound according to formula 1 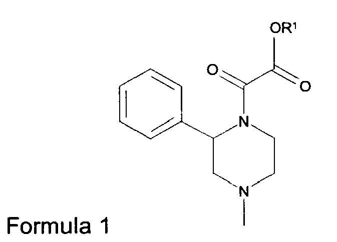 wherein R¹ is methyl, ethyl, n-propyl, isopropyl, benzyl or 2-haloethyl (such as 2-chloro-ethyl and 2,2,2-trifluorethyl), which compound is unique for use in a novel method to prepare separate (S)- and (R)-1-methyl-3-phenylpiperazine by enzymatic hydrolysis of such a compound. Although it was observed by Hu et al that the protease of Streptomyces griseus was less useful for a phenylpiperidine, this invention provides for a method to prepare (S)- and (R)-1-methyl-3-phenylpiperazine by enzymatic hydrolysis of the compound defined above, followed by separation of the hydrolysis product and cleavage of the oxalamic groups, whereby the protease of Streptomyces griseus is used as enzyme for the enzymatic hydrolysis.

The starting compound can be used as a (C1-C3)alkyl-, benzyl- or 2-haloethyl-oxalamate, of racemic 1-methyl-3-phenylpiperazine or any degree optically active mixture in order to obtain (S)- or (R)-1-methyl-3-phenylpiperazine of high optically active purity. (C1-C3)alkyl means methyl, ethyl, n-propyl and isopropyl.

Using an optimized protocol, (*S*)-1-methyl-3-phenylpiperazine can now be obtained in 36 % overall yield at 99.8 % ee and about 98 % purity.

The protease of Streptomyces griseus is an enzyme of the very large family of hydrolases. Hydrolases are able to perform reactions with water, but also in near anhydrous organic solvents. Some examples of hydrolases are lipases (hydrolysis of fats), proteases (hydrolysis of proteins) and esterases (hydrolysis of esters).

The use of the protease of Streptomyces griseus as hydrolase has a number of advantages. It is a relatively stable enzyme that can be stored as concentrated aqueous solutions or freeze dried powders. The enzyme does not require any cofactor, which, if needed would not only be economically unattractive, but many cofactors can be more fragile than the enzyme itself. The enzyme has a large active site and can handle the substrate needed for this reaction very well, despite the fact that small variations can give dramatic differences in reaction rate. Lastly, the enzyme has high stability in water/co-solvent mixtures or even neat organic solvents.

In biocatalysis the selectivity of a reaction is often expressed as the enantiomeric ratio or E. The enantiomeric ratio stands for the ratio between the initial reaction rates of the two enantiomers at equal concentration (t=0 for most reactions). The enantiomeric ratio can be calculated at any point in the reaction if two out the following three parameters are known: conversion, product ee and substrate ee. Under ideal circumstances the E is constant throughout the reaction. There are a number of assumptions in the formulas that are not always valid. Furthermore, at very high or very low conversion the E varies strongly with small variations of the conversion or ee. This means that the value becomes more sensitive to the accuracy of the measurement. Since selectivity is never really absolute, at 100% conversion you end up with a racemate again. This means that E values often seem to go down towards the very end of the reaction. Therefore, the E should be used as an indicative value and not as an absolute value. The following rules of thumb can often be used:
E = 1 No selectivity, equal rates for both enantiomers
E = 1-5 Low selectivity. High ee can only be reached if the undesired enantiomer is consumed in the reaction and then only at conversions >90%.
E = 5-25 Good possibilities for a process by consuming the wrong enantiomer. High product ee is only obtained at low conversion.
E = 25-100 High substrate as well as product ee at moderate conversions.
E > 100 Near absolute selectivity. The reaction often "stops" at 50% conversion (dramatic rate decrease). Possibilities for dynamic kinetic resolution to get 100% ee at 100% yield. Not only the substrate is obtained in high ee, but product displays high ee around theoretical (50 %) yield.

In a more specific embodiment the invention provides the method as defined above, whereby the hydrolysis is performed in a buffer free medium. The absence of a need to add a buffer to the reaction medium not only simplifies the method, but even improves the method by obtaining higher enantiomeric ratios. Without being bound to theory it is believed that the nitrogen at the 1 position in the piperazine ring contributes to this.

A more specific embodiment of the invention is to use in the method as defined above the methyloxalate of 1-methyl-3-phenylpiperazine in combination with a medium comprising toluene or methyl-t-butylether.

Another specific embodiment of the invention is to use in the method as defined above the ethyloxalate of 1-methyl-3-phenylpiperazine in combination with a medium comprising cyclohexane.

With the information in this description the skilled person may now further optimise the conditions for the method or find close alternatives of the method by selecting suitable media, concentrations and oxalamate esters.

### Examples

### Enzymatic hydrolysis of (m)ethyloxalamate derivates.

The racemic substrates were prepared by acylation of 1-methyl-3-phenylpiperazine using commercially available methyl chlorooxalate, yielding a crystalline oxalamate that could be purified by recrystallisation. A range of commercially available proteases were tested (table 1). Only esperase showed any activity, as observed by enrichment of one of the enantiomers. The absolute configuration was determined by comparison with a confirmed sample of the (S)-enantiomer. A short screen for reaction conditions was conducted (table 2). The E values were calculated on the basis of the conversion and the ee of the starting material and the product. The conversion was estimated using a small impurity in the starting material (already present in the starting piperazine) as an internal standard.

**Table 1: Screen of proteases in resolution of the ethyloxalamate**

| | enzyme | ee (43h) | ee (65h) | ee 85 h |
|---|---|---|---|---|
| 1 | none | 0 | n.d. | - |
| 2 | 0.1 ml esperase | 5 % | 13 % (R) | 21 % (R) |
| 3 | 0.1 ml everlase | < 1 % | < 1 % | - |
| 4 | 250 mg polarzyme | < 1 % | 1 % | - |
| 5 | 25 mg savinase CLEA | 2 % | 3 % | - |
| 6 | 25 mg alcalase CLEA | 4 % | 6 % | - |
| 7 | 25 mg proteinase N (fluka) | < 1 % | 1 % | - |

Conditions: 7 vials were filled with enzyme, 1 ml oxamate solution (0.1 M 1-methyl-3-phenylpiperazine ethyloxamate in methyl-t-butylether (MTBE)) and 2 ml 0.1 M phosphate buffer (pH 7.3). Stirring for 3 days at rT, reaction 2 was stirred for another 20h at 40 °C. Analysis by chiral GC.

**Table 2: Short condition screen for esperase**

| | enzyme | buffer | Solvent | ee | Conv |
|---|---|---|---|---|---|
| 1 | 0.25 ml esperase | 0.5 M pH 9.0 | MTBE | 6 % | 3.3 % |
| 2 | 0.25 ml esperase | 0.1 M pH 9.0 | MTBE | 15 % | 0 % (?) |
| 3 | 0.25 ml esperase | 0.5 M pH 9.0 | CAN | 16.2 % | 17 % (R, E=10) |
| 4 | 0.25 ml esperase | 0.1 M pH 9.0 | CAN | 22 % | 40 % (R) |
| 5 | 0.25 ml esperase | 0.1 M pH 8.2 | MTBE | 13 % | 7 % |
| 6 | 0.25 ml esperase | 0.1 M pH 10 | MTBE | 16 % | 2 % |
| 7 | 1 ml esperase | 0.1 M pH 9.0 | MTBE | 23 % | < 0 |
| 8 | 1 ml alcalase | 0.1 M pH 9.0 | MTBE | 20 % | < 0 |

Conditions: 8 vials were filled with enzyme, 1 ml oxamate solution (0.1 M 1-methyl-3-phenylpiperazine ethyloxamate in MTBE or acetonitrile (ACN)) and 2 ml phosphate buffer. Stirring for 18h. Analysis by chiral GC.

Using crude methyloxalamate and relatively pure ethyloxalamate a wide protease screen was conducted (table 3). A prototype CLEA of esperase did not give better results. A range of other enzymes in toluene/bicarbonate buffer showed no selectivity at all (table 4).

**Table 3: More extensive protease screen for the (m)ethyloxalamate**

| | Enzyme | m | Starting material | GC (ee oxalamate) |
|---|---|---|---|---|
| 1 | Trypsin Novo | 50 mg | Me* | 0 % |
| 2 | Europa protease 2 | 50 mg | Me* | -3 % |
| 3 | Europa protease 7 | 50 mg | Me* | 0 |
| 4 | Europa protease 12 | 50 mg | Me* | 0 |
| 5 | Europa esterase 2 | 50 mg | Me* | -3 % |
| 6 | Esperase CLEA | 50 mg | Me* | 11 % |
| 7 | Esperase CLEA | 250 mg | Me* | 12 % |
| 8 | Alcalase CLEA | 50 mg | Me* | 15 % |
| 9 | Alcalase CLEA | 250 mg | Me* | 43 % (R) |
| 10 | Microb. protease Fluka | 10 mg | Me* | 37 % (R) |
| 11 | Subtilisine A | 10 mg | Me* | 11 % |
| 12 | Esperase CLEA | 250 mg | Et | 4 % |
| 13 | Alcalase CLEA | 250 mg | Et | 9 % |
| 14 | Microb. protease Fluka | 10 mg | Et | 6 % |
| 15 | Subtilisine A | 10 mg | Et | < 2 % |

Conditions: 15 vials were filled with enzyme, 1 ml oxamate solution (0.1 M 1-methyl-3-phenylpiperazine (m)ethyloxamate in MTBE) and 2 ml 0.1 M phosphate buffer (pH 9.0). Stirring for 18h. Analysis by chiral GC.

**Table 4: Screening of enzymes in toluene bicarbonate buffer**

| E9/ | Enzyme | pH end | ee remaining substrate |
|---|---|---|---|
| 1 | Alcalase CLEA | 8.0 | 0 |
| 2 | Esperase CLEA | 8.1 | 0 |
| 3 | Savinase CLEA | 8.1 | < 3 |
| 4 | Novo Subtilisine | 8.1 | < 2 |
| 5 | CaL-A CLEA | 8.1 | 0 |
| 6 | CaL-B CLEA | 8.1 | 3 |
| 7 | CR CLEA | 8.3 | 0 |
| 8 | Amano acylase | 8.4 | 2 |
| 9 | Acylase I | 8.4 | 2 |
| 10 | Alcalase CLEA (pH 8 K-phosphate buffer) | 7.6 | 1 |

Conditions to Table 4: the dry enzymes (50 mg) were mixed with 1 ml stock solution (1-methyl-3-phenylpiperazine methyloxalamate (16 g) as impure, crude sirup was dissolved in 600 ml toluene) and 1 ml of 0.2 M bicarbonate buffer. Reaction at rT for 22h.

A good result was obtained with Streptomyces griseus protease. Table 5 shows the results; under the right conditions an excellent ee could be obtained for the desired enantiomer.

The best results were obtained in a buffer free medium, which used the extra nitrogen in the substrate as base. Even at a pH << 7 very good results were obtained for this protease.

**Table 5: Screen of conditions for S. griseus protease and ethyloxalamate**

| | Solvent | pH end | ee | E estim*. |
|---|---|---|---|---|
| 1 | 90 % ACN/water | 7.3 | 0 | - |
| 2 | 10 % ACN/water | 6.4 | 100%(S) | E=60 |
| 3 | 50 % ACN/water | 6.7 | 31 % (S) | - |
| 4 | 50 % dioxaan/water | 6.6 | 29 % (S) | - |
| 5 | 50 % t-BuOH/water | 5.9 | 90 % (S) | E=27 |
| 6 | 50 % MTBE/water | 5.9 | ca 98 % (S) | E=500 |
| 7 | 50 % MTBE/pH 8 0.1 M buffer | 7.1 | 50 % (S) | E =7 |

Conditions: 7 vials were filled with 8 mg protease Streptomyces griseus, 28 mg (0.1 mmol) oily ethyl oxalamate and 2 ml of the indicated solvent. Stirring for 20h at rT. Analysis by chiral GC. * Conversion estimated using the 3.8m impurity.

The enzyme was further tested in a range of conditions using (now pure) methyloxalamate and ethyloxalamate (table 6). Using co-solvent free conditions the methyloxalamate solidified, resulting in a thick suspension with obvious diffusion limitations that hampered complete optical purity at relatively high conversion. Only a small amount of the enzyme was needed.

The 2 esters showed differences in optimal conditions for resolution.

**Table 6: Further conditions screen of S. griseus protease and (m)ethyloxalamate**

| | ester | mg enzyme | Solvent | pH end | ee (all S) | E estim.* |
|---|---|---|---|---|---|---|
| 1 | Me | 0.1 | H₂O | 6.2 | 24 % | high |
| 2 | Me | 1 | H₂O | 6.0 | 97.4 % | 60 |
| 3 | Me | 10 | H₂O | 6.0 | 95.2 % | high |
| 4 | Me | 1 | 5% ACN | 6.2 | 87 % | 9 |
| 5 | Me | 1 | 5 % t-BuOH | 6.0 | 89 % | 12 |
| 6 | Me | 1 | 5 % acetone | 6.1 | 77 % | 12 |
| 7 | Me | 1 | MTBE | 5.6 | 100 % | high |
| 8 | Et | 0.1 | H₂O | 6.3 | 91 % | 8.6 |
| 9 | Et | 1 | H₂O | 6.2 | 100 % | >21 |
| 10 | Et | 10 | H₂O | 6.1 | 100 % | > 12 |
| 11 | Et | 1 | 5% ACN | 6.4 | 95.1 % | 12 |
| 12 | Et | 1 | 5 % acetone | 6.3 | 95.6 % | 9 |
| 13 | Et | 1 | MTBE | 6.0 | 49 % | slow? 5 |

Conditions: 13 vials were filled with Streptomyces griseus protease, 26/28 mg (0.1 mmol) oily (m)ethyl oxalamate and 2 ml of the indicated solvent. Stirring for 64h at rT. Analysis by chiral GC after basification. * Conversion estimated using the 3.8m impurity.

A further range of commercially available proteases were tested (table 7). For the 2 esters the optimal conditions from table 6 were used; methyl ester (now a solid) in a biphasic MTBE mixture, the ethyl ester as a suspended oil in pure water. During this experiment the oily ethyloxalamate also started to solidify, which could mean that the promising results of table 6 (exp 8) would not be reproducible.

In the enzyme screen only the ethyl ester gave two possible candidates, these were further tested at more realistic enzyme loading (table 8), albeit with little success. The addition of a small amount of acetic acid to improve solubility of the solid substrate was not successful, even though the enzyme seems to work at quite low pH.

**Table 7: Further protease screen for (m)ethyl oxalamate**

| | ester | Enzyme | Solvent | pH end | ee |
|---|---|---|---|---|---|
| 1 | Me | 2.8 mg S. griseus protease | MTBE* | 6.5 | 100 % (S) |
| 2 | Me | 10 mg Fluka Subtilisine A | MTBE | 6.3 | 0 |
| 3 | Me | 10 mg Fluka bact. Protease | MTBE | 6.0 | ca 5% (R) |
| 4 | Me | 25 mg proteinase A. melleus | MTBE | 6.4 | 0 |
| 5 | Me | 10 mg protease B. polymyxa | MTBE | 7.2 | 0 |
| 6 | Me | 10 mg protease A. saitoi | MTBE | 6.5 | 0 |
| 7 | Me | 100µl B. amyliquefaciens | MTBE | 6.1 | 0 |
| 8 | Me | 100µl A. oryzae protease | MTBE | 5.9 | ca. 5 % (S) |
| 9 | Me | 100µl Esperase | MTBE | 6.4 | 0 |
| 10 | Et | 2.8 mg S. griseus protease | H2O* | 6.5 | 100 % (S) |
| 11 | Et | 10 mg Fluka Subtilisine A | H2O | 6.8 | 0 |
| 12 | Et | 10 mg Fluka bact. Protease | H2O | 6.6 | 11 % (S) |
| 13 | Et | 25 mg proteinase A. melleus | H2O | 6.5 | 56 % (S) |
| 14 | Et | 10 mg protease B. polymyxa | H2O | 7.0 | 0 |
| 15 | Et | 10 mg protease A. saitoi | H2O | 7.0 | 0 |
| 16 | Et | 100µl B. amyliquefaciens | H2O | 6.5 | 0 |
| 17 | Et | 100µl A. oryzae protease | H2O | 6.4 | 57 % (S) |
| 18 | Et | 100µl Esperase | H2O | 6.8 | 0 |

Conditions: 18 vials are filled with 28 mg oily ethyl oxalamate or 1 ml of a 0.1 M solution of the methyl oxalamate in MTBE/5%isopropanol (otherwise no solubility). Water is added to 2 ml reaction volume (taking into account the volume of the enzyme). Indicated amount of enzyme. *Exp 1 and 10 contain 1 ml 0.1 M pH 8 tris buffer.

**Table 8: Screen of 2 other protease candidates in ethyl oxalamate**

| | enzyme | Acid | end | ee |
|---|---|---|---|---|
| 1 | 2.5 mg Amano protease M | 0 | suspension | 0 |
| 2 | (A. melleus) | 0.1 eq HOAc | suspension | 0 |
| 3 | | 0.2 eq HOAc | suspension | 0 |
| 4 | | 0.35 eq HOAc | suspension | -3 % |
| 5 | | 0.5 eq HOAc | clear | +3 % |
| 6 | 10µl A. Oryzae protease | 0 | suspension | -2 % |
| 7 | | 0.1 eq HOAc | suspension | 3 % |
| 8 | | 0.2 eq HOAc | suspension | 3 % |
| 9 | | 0.35 eq HOAc | suspension | 0 |
| 10 | | 0.5 eq HOAC | suspension | 0 |

Conditions: 10 vials were filled with 28 mg oily ethyl oxalamate. 1 ml water was added. Indicated amount of acetic acid to improve solubility of substrate.

### Scale-up of S. griseus protease catalysed reaction.

The main aim was to lower the loading of the enzyme and increase the substrate concentration without compromising on the final enantiopurity of the product. During the reactions in unbuffered water, the pH dropped considerable to well outside the optimum pH of the enzyme. So the first test was a pH screen, using a pH stat while comparing it to the unbuffered reaction (table 9). A reaction without pH control showed a higher ee in an earlier stage of the reaction. The ee of the isolated oxalamic acid product (R-enantiomer) was also higher, indicating a much more selective reaction (higher E). The same effects were seen with the ethyloxalamate, but solidification of the substrate complicated the comparison of the results a bit (table 10).

**Table 9: Effect of pH-control on methyl oxalamate**

| | pH control | ee (16h) | pH (40h) | ee (40h) | Yield | ee(R-prod) | E |
|---|---|---|---|---|---|---|---|
| A | pH stat 7.0 | 74 % | 6.6 | 100 % | 42 % | -71 % | 48 |
| B | none | 87 % | 5.5 | 100% | 47 % | -84 % | 68 |

Conditions: 10 mmol solid 1-methyl-3-phenylpiperazine methyloxalamate, 25 ml water, 25 ml MTBE, 50 mg S. griseus protease (2 wt%). Exp A pH control during the first 24h of reaction.

**Table 10: Effect of pH-control on ethyl oxalamate**

| | pH control | start pH | ee (S) | ee (R) | E |
|---|---|---|---|---|---|
| A | none | 8.0 | 99.7 | -92 % | 155 |
| B | none | 5.9 (HOAc) | 99.2 | -91 % | 112 |
| C | pH stat 7.0 | 8.1 | 99.3 | -79 % | 45 |

Conditions: 10 mmol oily 1-methyl-3-phenylpiperazine ethyloxalamate, 50 ml water, 50 mg S. griseus protease (2 wt%). Exp C pH control during the first 24h of reaction.

A final solvent screen was repeated using the best conditions known at much higher concentration than before. As both oxalamates had solidified a cosolvent was needed for both. Surprisingly, the optimum conditions were again not identical (table 11).

**Table 11: Final solvent screen for the (m)ethyl oxalamates**

| | Start material | Solvent | T | pH | ee (all S) |
|---|---|---|---|---|---|
| 1 | methyl | toluene | 21h | | 99.3 % |
| | | | 24h | 5.3 | 99.85 % |
| 2 | methyl | MTBE | 21h | | 94 % |
| | | | 24h | 5.5 | 48 % (!) |
| 3 | methyl | cyclohexane | 21h | | 39 % |
| | | | 24h | 5.6 | 82 % |
| 4 | ethyl | toluene | 21h | | 75 % |
| | | | 24h | 5.3 | 82 % |
| 5 | ethyl | MTBE | 21h | | 82 % |
| | | | 24h | 5.3 | 89 % |
| 6 | ethyl | cyclohexane | 21h | | 99.89 % |
| | | | 24h | 5.4 | 99.92 % |

Conditions: 2.6 or 2.8 g (10 mmol) solid (m)ethyl oxalamate, 10 ml water, 5 ml solvent, 50 mg (ca 2 wt%) S. griseus protease. Stirring at rT. 2,3 and 6 were suspensions. After 24h these were homogenised using EtOAc, pH adjusted to 9 and analysed using chiral GC.

As the selectivity for the ethyloxalamate was quite high, the melting point quite low and the described optimum temperature for S. griseus protease is quite high, a single higher temperature reaction was tried for the crude ethyloxalamate using a cyclohexane co-solvent.

At 50 °C 16^{h} reaction was sufficient for complete conversion to 99.8 % ee using only 1 wt% S. griseus protease. These conditions were used for making the following large scale sample.

### Preparation of final sample (S)-1-methyl-3-phenylpiperazine

A 170 g sample of the crude ethyl oxalamate was resolved using 1 wt% S. griseus protease in a 1 L vessel. The R-enantiomer of the ethyloxalamate ester was selectively hydrolyzed. After the reaction was stopped, the remaining S-enantiomer of the ethyloxalamate ester of 1-methyl-3-phenylpiperazine was obtained in a crude yield of 47 %. Hydrolysis of the ethyloxalamate ester by boiling in an excess 15 % HCl gave complete conversion to (S)-1-methyl-3-phenylpiperazine in 1h. Further work-up yielded quite a large quantity of insoluble precipitate of unknown composition. Extraction and Kugelrohr distillation of the product yielded yielded 42 g white solid (36 % overall; 99.8 % ee). The initial impurity of 0.5 % in the starting material was increased to 1.5 %.

A scale-up of much smaller magnitude using the recrystallised methyloxalamate provided a more pure sample, as the 0.5 % impurity in the starting material is not concentrated in the final product.

### Additional experimental details

### Materials

| | |
|---|---|
| Methyl chlorooxalate | Aldrich |
| Ethyl chlorooxalate | Acros |
| Trifluoroacetic anhydride | Acros |
| Acetic anhydride | Merck |
| Propionyl chloride | Aldrich |
| Butyroyl chloride | Aldrich |
| Benzoyl chloride | Aldrich |
| S. griseus protease | Sigma |
| Solvents | p.a. |

### Analyses

Samples were analysed on a chirasil-DEX CB GC column (helium carrier, 1:20 split). Temperature program: 140 °C for 2 min.; 5°C/ min to 180°C; 180 °C for 10 min.

The piperazine could not be resolved on the GC. Derivatisation as trifluoroacetamide was needed to achieve a good separation of the enantiomers. A small amount of piperazine (10-50 mg) was dissolved in CH₂Cl₂ and treated with triethylamine and trifluoroacetic anhydride. Basification after the reaction using 10 % sodium carbonate. The sample was dried before analysis.

TLC was performed using silica plates, CH₂Cl₂/MeOH mixtures (typically 90:10) as eluent and both UV fluoresence and I₂ detection.

### Synthesis of acylated racemates

### 1-Methyl-3-phenylpiperazine methyloxalamate

1-Methyl-3-phenylpiperazine (17.6 g; 0.1 mol) was dissolved in 100 dichloromethane. Triethylamine (5 ml; ca 0.03 mol) was added. A solution of methyl chlorooxalate (10 ml; 0.10 mol) in dichloromethane was slowly added under cooling. After the total addition a white suspension was formed. TLC showed complete conversion. The mixture was quenched with 10% sodium carbonate. The organic layer was washed again with carbonate, dried and evaporated to an oil (25.5 g; 97 %).

TLC: quite pure, some minor polar impurities. GC: chiral separation possible 15.7/16.0 min (contains ca. 0.4% of 3.8 min impurity (present in starting piperazine) that can be used as internal standard).

The material solidifies on standing. Attempt to recrystallise from CH₂Cl₂/hexane. This gives 20 g of light-brown solid (76 %). mp 103-5°C.

GC: 3.8 min impurity is removed.

### 1-Methyl-3-phenylpiperazine ethyloxalamate

1-Methyl-3-phenylpiperazine (123.2 g; 0.70 mol) was dissolved in 500 dichloromethane. Triethylamine (30 ml; ca 0.2 mol) was added. A solution of ethyl chlorooxalate (107 g; 0.78 mol) in dichloromethane was slowly added under cooling. At 2/3 of the total addition a thick suspension was formed. Even after addition of more solvent, stirring remained difficult. The mixture was quenched with 10% sodium carbonate. The organic layer is washed again with carbonate, dried and evaporated to an orange oil (191.2 g; 0.69 mol; 99 %). Crystallisation with seeding proved difficult. Deep evaporation and storage as oil.TLC: very pure, a small amount of coloured polar material on baseline. No trace of the dioxamide (prepared from oxalylchloride and piperazine). GC: 18.0/18.2 min, 0.36 area% of 3.8 min impurity. A small sample (20 g) was stirred with water to induce crystallisation. mp ca 45 °C. The main bulk of the oil solidified after a few days of standing. Melting was needed before use.

### Acetyl 1-methyl-3-phenylpiperazine

1-Methyl-3-phenylpiperazine (17.6 g; 0.1 mol) was dissolved in 100 dichloromethane. Acetic anhydride and triethylamine were added. Aqueous work-up yielded >100 % of smelly oil (excess Ac2O). Kugelrohr distillation at 160 °C/0.05 mbar yielded 20.6 g oil (94 %). Chiral GC: 10.3/10.6 min

### Propionyl 1-methyl-3-phenylpiperazine

1-Methyl-3-phenylpiperazine (17.6 g; 0.1 mol) was dissolved in 100 dichloromethane. Triethylamine (15 ml; 0.1 mol) was added. A solution of propionyl chloride (10 g; 0.11 mol) in dichloromethane was slowly added under cooling. After the total addition a white suspension was formed. The mixture was quenched with 10% sodium carbonate. The organic layer was washed again with carbonate, dried and evaporated to an oil (23.34g; 100 %). Kugelrohr distillation at 187 °C/0.05 mbar yielded 21.6 g oil (93 %). Chiral GC: 10.25/10.39 min

### Butyryl 1-methyl-3-phenylpiperazine

1-Methyl-3-phenylpiperazine (17.6 g; 0.1 mol) was dissolved in 100 dichloromethane. Triethylamine (5 ml; 0.05 mol) was added. A solution of butyroyl chloride (11.6 g; 0.11 mol) in dichloromethane was slowly added under cooling. After the total addition a white suspension was formed. The mixture was quenched with 10% sodium carbonate. The organic layer was washed again with carbonate, dried and evaporated to an oil (24g). Kugelrohr distillation of 22.5 g at >200 °C/0.05 mbar yielded 22.0 g oil (95 %). Chiral GC: 12.87/12.98 min, severe overlap.

### Benzoyl 1-methyl-3-phenylpiperazine

1-Methyl-3-phenylpiperazine (17.6 g; 0.1 mol) was dissolved in 100 dichloromethane. Triethylamine (15 ml; 0.1 mol) was added. A solution of benzoyl chloride (16 g; 0.114 mol) in dichloromethane was slowly added under cooling. After the total addition a white suspension was formed. The mixture was quenched with 10% sodium carbonate. The organic layer was washed again with carbonate, dried and evaporated to an oil (ca 30 g). Purification by silica filtration using CH₂Cl₂/MeOH (95:5). Evaporation of the appropriate fractions yielded 26.2 g oil (94 %) Chiral GC: no separation using various methods.

### Trifluoroacetyl 1-methyl-3-phenylpiperazine

1-Methyl-3-phenylpiperazine (1.8 g; 0.01 mol) was dissolved in 50 dichloromethane. Triethylamine (1 ml; 0.07 mol) was added. Trifluoroacetic anhydride (2 ml) was added neat. The mixture was quenched with 10% sodium carbonate. The organic layer was washed again with carbonate, dried and evaporated to an oil (2.5 g; 92 %). TLC very pure. Chiral GC: 5.9/6.2 min.

### Screening reactions

Screening reactions were conducted as described in footnotes of the tables. 5 ml vessels were used for 1-4 ml reactions, 30 ml vials for larger optimisation reactions. Reactions that ended up too acidic were neutralised to pH>8 to allow the extraction of the basic piperazine (-derivative).

### Sample preparation of (S)-1-methyl-3-phenylpiperazine

170 g (0.62 mol) solid ethyl oxalamate was molten and transferred into a 1 L flask. 180 ml cyclohexane and 700 ml water were added, followed by 1.7 g (1 wt%) S. griseus protease. Stirring at 50°C on a heating plate for 23h. Gas chromatography (GS) of top layer showed >99.9 % ee. The pH of 5.22 was adjusted to 9 using 1 M NaOH.

The reaction mixture was extracted three times with ethyl acetate. After drying and evaporation of the organic phase 80 g brown oil (47 %; E>160) was obtained. This (S)-ethyloxalamate ester (+ 4.7 g product of an earlier 10g scale resolution) was hydrolyzed by reflux in 400 ml 15% HCl (ca 2 mol) for 1 h. The hydrolysis was determined to be ca. 99.5 % by GC. The reaction mixture was cooled down and to the pH was adjusted to pH >11. The aqueous phase was extracted with 3x 250 ml CH₂Cl₂. A large amount of insoluble precipitate was formed after neutralisation and was removed by filtration. The organic extract was dried and evaporated to provide 41 g of (S)-1-methyl-3-phenylpiperazine as an oil. Further extraction of the water phase using ethyl acetate (100 ml), toluene and ether yielded another 6 g of (S)-1-methyl-3-phenylpiperazine (about 41 % overall yield). High vacuum distillation on the Kugelrohr (140°C/0.05 mbar) yielded 41.8 g colourless oil that crystallised after seeding (238 mmol; 36 % overall). Distillation residue weighed 0.8 g. The melting point of the product was 52°C and the ee was found to be 99.8%GC showed an increase of an impurity present in the starting racemate from 0.5% to 1.5 %.

## Claims

1. A compound according to formula 1, 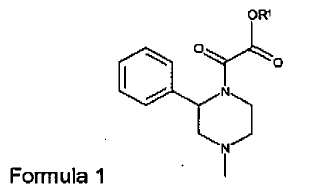 wherein R¹ is methyl, ethyl, n-propyl, isopropyl, benzyl or 2-haloethyl.

2. The oxalamic derivative of (R)-1-methyl-3-phenylpiperazine according to formula 2.

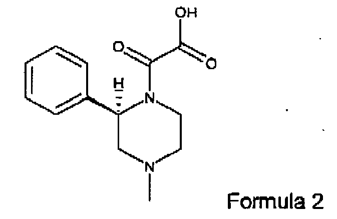

3. The oxalamate derivative of (S)-1-methyl-3-phenylpiperazine according to formula 3 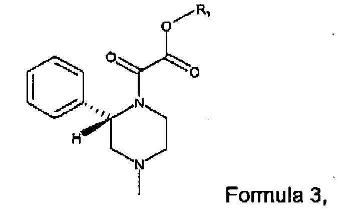 wherein R¹ is methyl, ethyl, n-propyl, isopropyl, benzyl or 2-haloethyl.

4. A method to prepare (S)-1-methyl-3-phenylpiperazine by enzymatic hydrolysis of the compound according to claim 1, followed by separation and cleavage of the oxalamic ester group from the reaction product, whereby the protease of Streptomyces griseus is used as enzyme for the enzymatic hydrolysis.

5. A method to prepare (R)-1-methyl-3-phenylpiperazine by enzymatic hydrolysis of the compound according to claim 1, followed by separation and cleavage of the oxalamic acid group from the reaction products, whereby the protease of Streptomyces griseus is used as enzyme for the enzymatic hydrolysis.

6. The method according to claim 4 or 5, **characterised in that** the hydrolysis is performed in a buffer free medium.

7. The method according to any one of claim 4-6, **characterised in that** the hydrolysis is of methyloxalate of 1-methyl-3-phenylpiperazlne and the medium for the hydrolysis comprises toluene or methyl-t-butylether.

8. The method according to any one of claim 4-6, **characterised in that** the hydrolysis is of ethyloxalate of 1-methyl-3-phenylpiperazine and the medium comprises cyclohexane.

## Patentansprüche

1. Verbindung gemäss Anspruch 1, 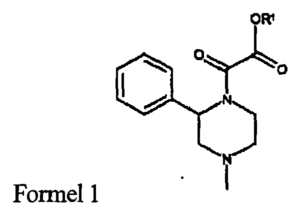 wobei R¹ Methyl, Ethyl, n-Propyl, Isopropyl, Benzyl oder 2-Haloethyl ist.

2. Oxalamisches Derivat von (R)-1-Methyl-3-Phenylpiperazin gemäss Formel 2:

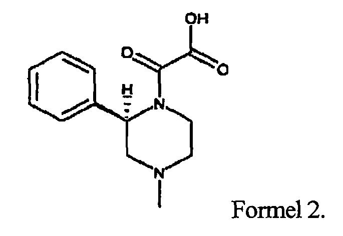

3. Oxalamat-Derivat von (S)-1-Methyl-3-Phenylpiperazin gemäss Formel 3: 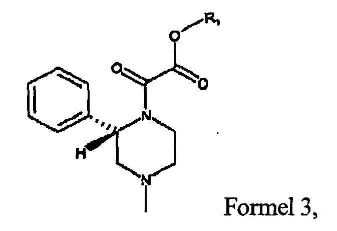 wobei R¹ Methyl, Ethyl, n-Propyl, Isopropyl, Benzyl oder 2-Haloethyl ist.

4. Verfahren zur Herstellung von (S)-1-Methyl-3-Phenylpiperazin durch enzymatische Hydrolyse der Verbindung gemäss Anspruch 1, gefolgt von Trennung und Abspaltung der oxalamischen Ester-Gruppe aus dem Reaktionsprodukt, wobei die Protease des Streptomyces griseus als Enzym für die enzymatische Hydrolyse eingesetzt wird.

5. Verfahren zur Herstellung von (R)-1-Methyl-3-Phenylpiperazin durch enzymatische Hydrolyse der Verbindung gemäss Anspruch 1, gefolgt von Trennung und Abspaltung der oxalamischen Säure-Gruppe aus den Reaktionsprodukten, wobei die Protease des Streptomyces griseus als Enzym für die enzymatische Hydrolyse eingesetzt wird.

6. Verfahren gemäss Anspruch 4 oder 5, **dadurch gekennzeichnet, dass** die Hydrolyse in einem pufferfreien Medium durchgeführt wird.

7. Verfahren gemäss irgendeinem der Ansprüche 4 bis 6, **dadurch gekennzeichnet, dass** die Hydrolyse vom Methyloxalat von 1-Methyl-3-Phenylpiperazin ist und das Medium für die Hydrolyse Toluen oder Methyl-t-Butylether umfasst.

8. Verfahren gemäss irgendeinem der Ansprüche 4 bis 6, **dadurch gekennzeichnet, dass** die Hydrolyse vom Ethyloxalat von 1-Methyl-3-Phenylpiperazin ist und das Medium Cyclohexan umfasst.

## Revendications

1. Un composé selon la formule 1. 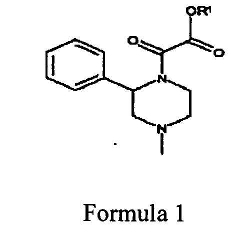 dans laquelle R¹ est un radical méthyl, éthyl, n-propyl, isopropyl, benzyl ou 2-haloéthyl.

2. Le dérivé oxalamique de la (R)-1-méthyl-3-phénylpiperazine selon la formule 2.

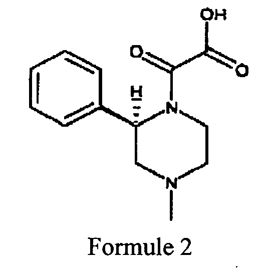

3. L'oxalamate de (S)-1-méthyl-3∼phénylpiperazine selon la formule 3 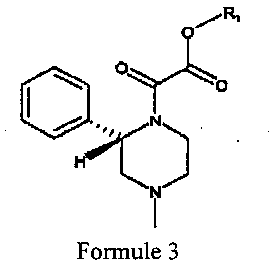 dans laquelle R¹ est un radical méthyl, éthyl, n-propyl, isopropyl, benzyl ou 2-haloéthyl.

4. Un procédé pour préparer la (S)-1-méthyl-3-phénylpiperazine par hydrolyse enzymatique du composé selon la revendication 1, suivié d'une séparation et d'un clivage du groupe ester oxalamique du milieu réactionnel, procédé dans lequel la protéase de Streptomyces griseus est employée en tant qu'enzyme pour l'hydrolyse enzymatique.

5. Un procédé pour preparer la (R)-1-méthyl-3-phénylpipernzine par hydrolyse enzymatique du composé selon la revendication 1, suivie d'une séparation et d'un clivage du groupe acide oxalamique du milieu réactionnel, procédé dans lequel la protéase de Streptomyces griseus est employée en tant qu'enzyme pour l'hydrolyse enzymatique..

6. Le procédé selon la revendication 4 ou 5, **caractérisé en ce que** l'hydrolyse est mise en oeuvre dans un milieu exempt de tampon.

7. Le procédé selon une quelconque des revendications 4 à 6, **caractérisé en ce que** l'hydrolyse est celle du méthyloxalate de 1-méthyl-3-phénylpiperazlne et le milieu pour l'hydrolyse comprend du toluène ou du méthyl-t-butyléther.

8. Le procédé selon une quelconque des revendications 4 à 6, **caractérisé en ce que** l'hydrolyse est celle de l'éthyloxalate de 1-méthyl-3-phénylpiperazine et le milieu pour l'hydrolyse comprend du cyclohexane.
